(19) Europäisches Patentamt
European Patent Office
Office européen des brevets

(11) Veröffentlichungsnummer : **0 164 056**
**B1**

(12) # EUROPÄISCHE PATENTSCHRIFT

(45) Veröffentlichungstag der Patentschrift : **07.09.88**

(21) Anmeldenummer : **85106547.4**

(22) Anmeldetag : **29.05.85**

(51) Int. Cl.[4] : **C 07 D335/02**, C 07 D409/12, C 07 D413/12, C 07 D417/12, A 01 N 43/18

(54) **Substituierte Tetrahydrothiopyran-2,4-dione.**

(30) Priorität : **08.06.84 DE 3421351**

(43) Veröffentlichungstag der Anmeldung : **11.12.85 Patentblatt 85/50**

(45) Bekanntmachung des Hinweises auf die Patenterteilung : **07.09.88 Patentblatt 88/36**

(84) Benannte Vertragsstaaten : **AT BE CH DE FR GB IT LI NL**

(56) Entgegenhaltungen :
**EP-A- 0 071 707**
**DE-A- 2 317 987**
**DE-B- 2 439 104**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Wroblowsky, Heinz-Jürgen, Dr.**
**Gladbacher Strasse 34**
**D-4018 Langenfeld (DE)**
Erfinder : **Stetter, Jörg, Dr.**
**Gellertweg 4**
**D-5600 Wuppertal 1 (DE)**
Erfinder : **Eue, Ludwig, Dr.**
**Paul-Klee-Strasse 36**
**D-5090 Leverkusen 1 (DE)**
Erfinder : **Schmidt, Robert R., Dr.**
**Im Waldwinkel 110**
**D-5060 Bergisch-Gladbach 2 (DE)**
Erfinder : **Santel, Hans-Joachim, Dr.**
**Gerstenkamp 19**
**D-5000 Koeln 80 (DE)**

**EP 0 164 056 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue substituierte Tetrahydrothiopyran-2,4-dione, ein Verfahren zu ihrer Herstellung und ihre Verwendung als Herbizide.

Es ist bereits bekannt, daß bestimmte α-Pyrone, wie beispielsweise das 3-(1-Allyloximino-ethyl)-4-hydroxy-6-methyl-α-pyron, herbizide Eigenschaften besitzen (vgl. z. B. Jap. Pat. 53/34776 oder DE-A-23 17 987 oder DE-B-24 39 104).

Die Wirkung dieser Verbindungen ist jedoch insbesondere bei niedrigen Aufwandmengen und -konzentrationen nicht immer in allen Anwendungsbereichen völlig zufriedenstellend.

Es wurden neue Tetrahydrothiopyran-2,4-dione der Formel (I),

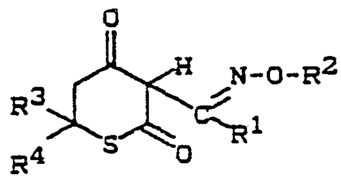

(I)

in welcher

$R^1$ für Wasserstoff, Methyl, Ethyl, n- oder i-Propyl, n-, i-, s- oder t-Butyl, n- oder i-Pentyl, n- oder i-Hexyl, Vinyl, Allyl, n- oder i-Butenyl, n- oder i-Pentenyl, n- oder i-Hexenyl, Propargyl, Methylpropargyl, Dimethylpropargyl, Methoxymethyl, Ethoxymethyl, Isopropoxymethyl, Methoxyethyl, Ethoxyethyl, Methylthiomethyl, Methylthioethyl, Ethylthioethyl, Trifluormethyl, Dichlorfluormethyl, Difluorchlormethyl, Trichlormethyl, Dichlormethyl, Chlormethyl, 2-Chlorethyl, 2-Bromethyl, 2,2,2-Trichlorethyl, 2,2,2-Trifluorethyl, Cyclopropyl, Cyclobutyl, Cyclopentyl, Cyclohexyl oder gegebenenfalls ein- bis dreifach, gleich oder verschieden durch Fluor, Chlor, Brom, Cyano, Nitro, Methyl, Methoxy oder Trifluormethyl substituiertes Phenyl steht,

$R^2$ für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, Methoxymethyl, Ethoxymethyl, Isopropoxymethyl, Methoxyethyl, Ethoxyethyl, Methylthiomethyl, Methylthioethyl, Ethylthioethyl, 2-Chlorethyl, 2-Bromethyl, 2,2,2-Trifluorethyl, 2,2,2-Trichlorethyl, 3-Chlorpropenyl, Methoxycarbonylmethyl, Ethoxycarbonylmethyl, Methoximinomethyl, Methoximinoethyl, Methoximino-2-propyl, gegebenenfalls ein- bis dreifach, gleich oder verschieden durch Fluor, Chlor, Brom, Cyano, Nitro, Methyl, Methoxy oder Trifluormethyl substituiertes Benzyl oder für einen gegebenenfalls ein- bis dreifach, gleich oder verschieden durch Chlor, Methyl oder Trifluormethyl substituierten über eine Methylenbrücke verknüpften Heterocyclus der Formel

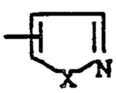 ; 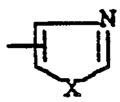 ; 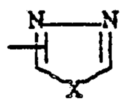 oder 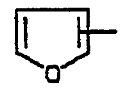

steht, wobei X jeweils für Sauerstoff oder Schwefel steht, und

$R^3$ und $R^4$ unabhängig voneinander jeweils für Wasserstoff, Methyl, Ethyl, n- oder i-Propyl, n-, i-, s- oder t-Butyl, Methoxymethyl, Ethoxymethyl, Methoxyethyl, Ethoxyethyl, Methylthiomethyl, Ethylthiomethyl, Ethylthioethyl, Ethylthiopropyl, Cyclopentyl, Cyclohexyl oder für jeweils gegebenenfalls ein- bis dreifach, gleich oder verschieden durch Fluor, Chlor, Brom, Cyano, Nitro, Methyl, Methoxy, Ethoxy, Methylthio, Ethylthio oder Trifluormethyl substituiertes Phenyl, Benzyl, Phenylethyl, Phenoxymethyl oder Phenoxyethyl stehen.

Die erfindungsgemäßen Verbindungen der Formel (I) liegen im tautomeren Gleichgewicht vor mit Verbindungen der Formeln (Ia), (Ib) und (Ic),

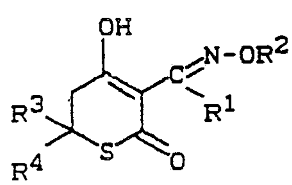

(Ia)

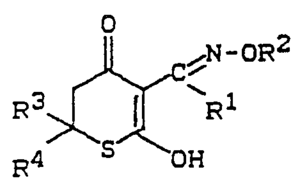

(Ib)

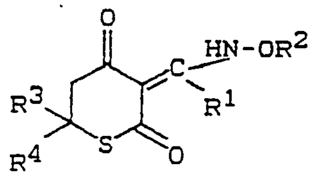

(Ic)

in welcher $R^1$, $R^2$, $R^3$ und $R^4$ die oben angegebene Bedeutung haben.

Besonders die Enolformen (Ia) und (Ib) und die Enaminstruktur (Ic) werden durch starke intramolekulare Wasserstoffbrückenbindungen stabilisiert.

Außerdem können die Verbindungen der Formel (I) als geometrische und/oder optische Isomere oder Isomerengemische unterschiedlicher Zusammensetzung vorliegen. Sowohl die reinen Isomeren als auch die Isomerengemische, ebenso wie die verschiedenen tautomeren Strukturen werden erfindungsgemäß beansprucht.

Weiterhin wurde gefunden, daß man die neuen Tetrahydrothiopyran-2,4-dione der allgemeinen Formel (I) erhält, wenn man Aldehyde bzw. Ketone der Formel (II),

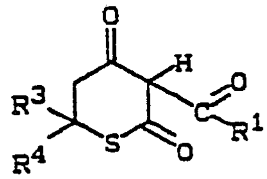

(II)

in welcher $R^1$, $R^3$ und $R^4$ die oben angegebene Bedeutung haben, mit Hydroxylamin-Derivaten der Formel (III),

$$H_2N—O—R^2 \qquad (III)$$

in welcher $R^2$ die oben angegebene Bedeutung hat, oder mit deren Hydrosalzen, gegebenenfalls in Gegenwart eines Verdünnungsmittels und gegebenenfalls in Gegenwart eines Säurebindemittels, umsetzt.

Schließlich wurde gefunden, daß die neuen substituierten Tetrahydrothiopyran-2,4-dione der Formel (I) herbizide, insbesondere auch selektiv-herbizide Eigenschaften besitzen.

Überraschenderweise zeigen die neuen substituierten Tetrahydrothiopyran-2,4-dione der Formel (I) eine erheblich bessere herbizide Wirksamkeit als die aus dem Stand der Technik bekannten α-Pyron-Derivate, wie beispielsweise das 3-(1-Allyloximino-ethyl)-4-hydroxy-6-methyl-α-pyron, welche chemisch und wirkungsmäßig naheliegende Verbindungen sind.

Die erfindungsgemäßen substituierten Tetrahydrothiopyran-2,4-dione sind durch die Formel (I) allgemein definiert.

Im einzelnen seien außer den bei den Herstellungsbeispielen genannten Verbindungen die folgenden Tetrahydrothiopyran-2,4-dione der allgemeinen Formel (I) genannt:

Tabelle 1

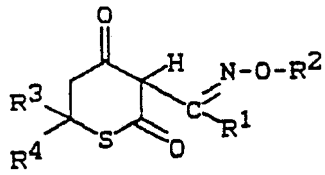

(I)

| $R^1$ | $R^2$ | $R^3$ | $R^4$ |
|---|---|---|---|
| $-CH_3$ | $-C_2H_5$ | $-CH_3$ | $-CH_3$ |
| $-CH_3$ | $-CH_2-CH=CH_2$ | $-CH_3$ | $-CH_3$ |
| $-CH_3$ | $-CH_2-CH=CH_2$ | -H | 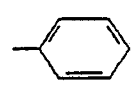 |

Tabelle 1 (Fortsetzung)

| $R^1$ | $R^2$ | $R^3$ | $R^4$ |
|---|---|---|---|
| $-C_2H_5$ | $-C_2H_5$ | $-CH_3$ | $-CH_3$ |
| $-C_2H_5$ | $-CH_2-CH=CH_2$ | $-CH_3$ | $-CH_3$ |
| $-C_2H_5$ | $-CH_2-CH=CH_2$ | $-CH_3$ | 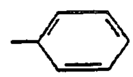 |
| $-C_3H_7$ | $-CH_3$ | $-CH_3$ | $-CH_3$ |
| $-C_3H_7$ | $-C_2H_5$ | $-CH_3$ | $-CH_3$ |
| $-C_3H_7$ | $-CH(CH_3)_2$ | $-CH_3$ | $-CH_3$ |
| $-C_3H_7$ | $-C_2H_5$ | $-H$ | 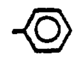 |
| $-C_3H_7$ | $-CH_2-CH=CH_2$ | $-CH_3$ | $-CH_3$ |
| $-C_3H_7$ | $-CH_2-CH=CH_2$ | $-H$ | 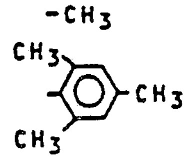 |
| $-C_3H_7$ | $-CH_2-C\equiv CH$ | $-CH_3$ | $-CH_3$ |
| $-C_3H_7$ | 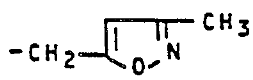 | $-CH_3$ | $-CH_3$ |
| $-C_3H_7$ | 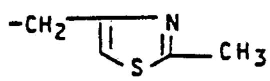 | $-CH_3$ | $-CH_3$ |
| $-C_3H_7$ | $-C_2H_5$ | $-H$ | 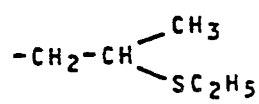 |
| $-C_3H_7$ | $-C_2H_5$ | $-H$ | 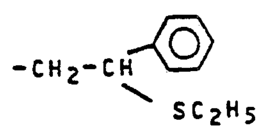 |
| $-C_3H_7$ | $-CH_2-CH=CH_2$ | $-H$ | 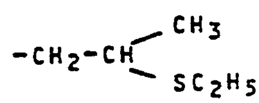 |
| $-C_3H_7$ | $-CH_2-CH=CH_2$ | $-H$ | 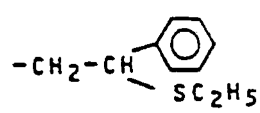 |
| $-C_3H_7$ | $-C_2H_5$ | $-H$ | 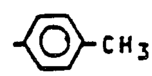 |
| $-C_3H_7$ | $-C_2H_5$ | $-H$ | 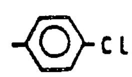 |
| $-C_3H_7$ | $-CH_2-CH=CH_2$ | $-H$ | 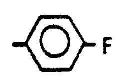 |
| $-C_3H_7$ | 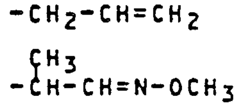 | $-CH_3$ | $-CH_3$ |

Tabelle 1 (Fortsetzung)

| $R^1$ | $R^2$ | $R^3$ | $R^4$ |
|---|---|---|---|
| $-C_3H_7$ | $-CH_2-CH=N-OCH_3$ | $-CH_3$ | $-CH_3$ |
| $-C_3H_7$ | $-\overset{CH_3}{CH}-CH=N-OCH_3$ | $-H$ | 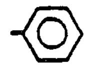 |
| $-C_3H_7$ | $-CH_2-CH=CH-Cl$ | $-CH_3$ | $-CH_3$ |
| $-C_3H_7$ | $-C_2H_5$ | $-H$ | 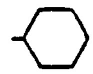 |
| $-C_3H_7$ | $-CH_2-CH=CH_2$ | $-H$ | 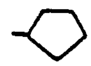 |
| $-C_3H_7$ | $-CH_2-O-CH_3$ | $-CH_3$ | $-CH_3$ |
| $-C_3H_7$ | $-CH_2-S-CH_3$ | $-CH_3$ | $-CH_3$ |
| $-C_3H_7$ | $-CH_2-$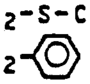 | $-CH_3$ | $-CH_3$ |

Verwendet man beispielsweise 3-Butyryl-6,6-dimethyltetrahydrothiopyran-2,4-dion und O-Allylhydroxylamin als Ausgangsstoffe, so läßt sich der Reaktionsablauf des erfindungsgemäßen Verfahrens durch das folgende Formelschema darstellen :

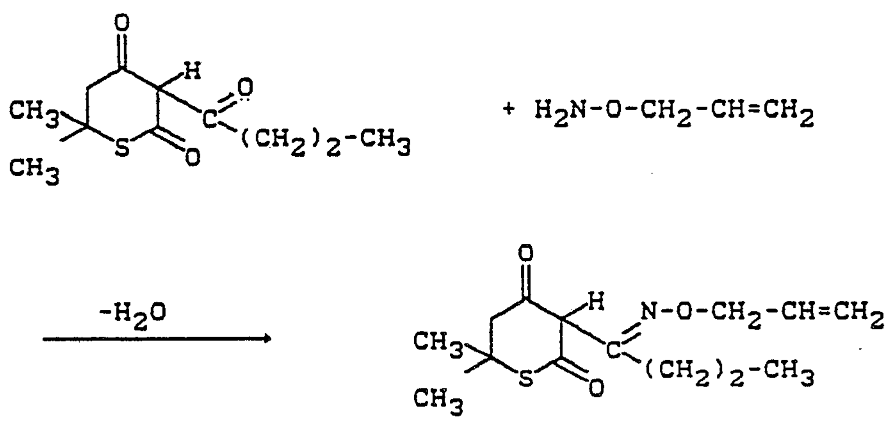


Die Aldehyde bzw. Ketone der Formel (II) sind noch nicht bekannt. Man erhält sie, wenn man Tetrahydrothiopyran-2,4-dione der Formel (IV),

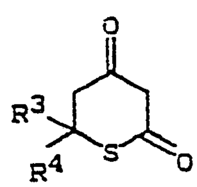

(IV)

in welcher $R^3$ und $R^4$ die oben angegebene Bedeutung haben, mit Acylierungsmitteln der Formel (V),

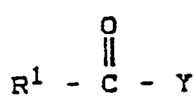

(V)

in welcher

$R^1$ die oben angegebene Bedeutung hat und

Y für eine elektronenanziehende Abgangsgruppe, insbesondere für Halogen oder den Rest

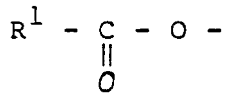

steht, wobei $R^1$ die oben angegebene Bedeutung hat, entweder zunächst in einer ersten Stufe, gegebenenfalls in Gegenwart eines Verdünnungsmittels (wie beispielsweise Toluol) und gegebenenfalls in Gegenwart eines Säurebindemittels (wie beispielsweise Diazabicycloundecen (DNU)), bei Temperaturen zwischen — 20 °C und + 50 °C umsetzt und die so erhältlichen O-Acylderivate der Formel (VI),

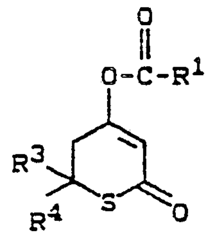

(VI)

in welcher $R^1$, $R^3$ und $R^4$ die oben angegebene Bedeutung haben, in einer 2. Stufe, ebenfalls gegebenenfalls in Gegenwart eines Verdünnungsmittels (wie beispielsweise Toluol) und in Gegenwart eines basischen Katalysators (wie beispielsweise 4-(N,N-Dimethylamino)-pyridin), bei Temperaturen zwischen 0 °C und + 120 °C umlagert, oder in einem Reaktionsschritt, ebenfalls in Gegenwart eines Verdünnungsmittels (wie beispielsweise Pyridin) und gegebenenfalls in Gegenwart eines Katalysators (wie beispielsweise Zinkchlorid), bei Temperaturen zwischen 0 °C und + 150 °C umsetzt.

Die Tetrahydrothiopyran-2,4-dione der Formel (IV) sind teilweise bekannt [vgl. Monatshefte für Chemie 113, 1283-1297 (1982)].

Man erhält die noch nicht bekannten Vertreter in prinzipiell bekannter Weise, so zum Beispiel, wenn man Methyl-Vinyl-Ketone der Formel (VII),

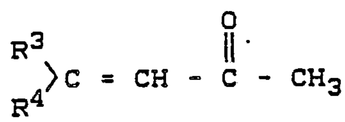

(VII)

in welcher $R^3$ und $R^4$ die oben angegebene Bedeutung haben, mit Diethylammonium N,N-Diethyldithiocarbamat der Formel (VIII),

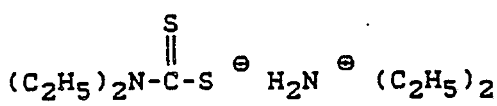

(VIII)

gegebenenfalls in Gegenwart eines Verdünnungsmittels (wie beispielsweise Brombenzol) bei Temperaturen zwischen + 50 °C und + 200 °C cyclisiert und die so erhältlichen Diethylaminoverbindungen der Formel (IX),

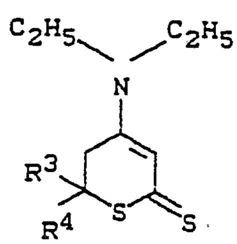

(IX)

in welcher $R^3$ und $R^4$ die oben angegebene Bedeutung haben, zunächst mit wäßrigem Natriumhydroxid bei Temperaturen zwischen + 30 °C und + 150 °C umsetzt und die so erhältlichen Thiopyranthionverbindungen der Formel (X),

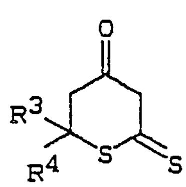

(X)

in welcher $R^3$ und $R^4$ die oben angegebene Bedeutung haben, anschließend mit wäßrigem Wasserstoffperoxid, gegebenenfalls in Gegenwart eines Verdünnungsmittels (wie beispielsweise Ethanol) und gegebenenfalls in Gegenwart eines basischen Katalysators (wie beispielsweise Kaliumhydroxid), bei Temperaturen zwischen — 30 °C und + 30 °C umsetzt.

Die Acylierungsmittel der Formel (V), die Methyl-Vinyl-Ketone der Formel (VII) und das Diethylammonium N,N-Diethyldithiocarbamat der Formel (VIII) sind allgemein bekannte Verbindungen der organischen Chemie.

Die Hydroxylamin-Derivate der Formel (III) und deren Hydrosalze sind ebenfalls allgemein bekannte Verbindungen der organischen Chemie.

Als Verdünnungsmittel zur Durchführung des erfindungsgemäßen Verfahrens kommen inerte organische Lösungsmittel in Frage. Vorzugsweise verwendet man Alkohole oder wäßrige Alkoholgemische, beispielsweise Methanol, Ethanol, n- und i-Propanol sowie deren Gemische mit Wasser.

Werden die Hydroxylamin-Derivate der Formel (III) in Form ihrer Salze — vorzugsweise verwendet man Hydrochloride — eingesetzt, so arbeitet man üblicherweise in Gegenwart eines Säurebindemittels. Als solche kommen alle üblichen anorganischen oder organischen Basen in Frage. Hierzu gehören beispielsweise alkalimetallhydroxide, wie Natriumhydroxid oder Kaliumhydroxid, Alkalimetallcarbonate, wie Natriumcarbonat, Kaliumcarbonat oder Natriumhydrogencarbonat, Alkalimetallacetate, wie Natriumacetat oder Alkalimetallalkoholate, wie Natrium- oder Kaliummethylat oder -ethylat. Die Reaktionstemperaturen können bei der Durchführung des erfindungsgemäßen Verfahrens in einem größeren Bereich variiert werden. Im allgemeinen arbeitet man zwischen 0 °C und + 150 °C, vorzugsweise zwischen + 20 °C und + 100 °C.

Bei der Durchführung des erfindungsgemäßen Verfahrens setzt man pro Mol an Aldehyd bzw. Keton der Formel (II) im allgemeinen 1,0 bis 2,0 Mol, vorzugsweise 1,0 bis 1,5 Mol an Hydroxylamin-Derivat der Formel (III) und gegebenenfalls 1,0 bis 2,0 Mol, vorzugsweise 1,0 bis 1,5 Mol an Säurebindemittel ein. Die Aufarbeitung und Isolierung der Reaktionsprodukte der Formel (I) erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Ortan aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden :

Dikotyle Unkräuter der Gattungen

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen, z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von monokotylen Unkräutern in dikotylen Kulturen wie beispielsweise in Zuckerrüben, Soja oder Baumwolle einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage : z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylaryl-polyglykolether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z. B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide ; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on oder bekannte Nitrodiphenylether zur Unkrautbekämpfung in Sojabohnen und bekannte Harnstoffe in Baumwolle oder Ackerbohnen in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

(Siehe Beispiel 1 Seite 9 f.)

## Herstellungsbeispiele

### Beispiel 1

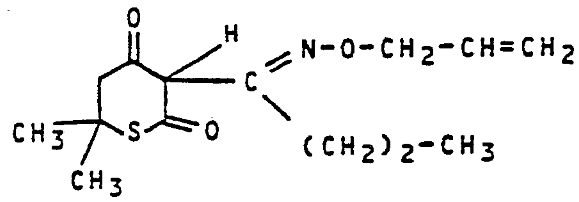

8,5 g (0,077 Mol) O-Allylhydroxylaminhydrochlorid in 50 ml Methanol werden unter Rühren und Eiskühlung mit 4,2 g (0,07 Mol) Natriummethylat versetzt und weitere 15 Minuten bei Raumtemperatur gerührt. Die Mischung wird filtriert und das Filtrat zu einer Lösung von 16 g (0,07 Mol) 6,6-Dimethyl-3-butyryltetrahydrothiopyran-2,4-dion gegeben. Die Reaktionsmischung wird bei Raumtemperatur bis zur vollständigen Umsetzung (Kontrolle mittels Dünnschichtchromatographie/Chloroform + 2 % Methanol/Kieselgel) gerührt, im Vakuum eingeengt, der Rückstand in Wasser aufgenommen, mit Chloroform extrahiert, die vereinigten organischen Phasen mit 5 %iger Salzsäure gewaschen, über Natriumsulfat getrocknet und im Vakuum vom Lösungsmittel befreit. Man erhält 15,8 g (80 % der Theorie) an 3-(1-Allyloximino-butyl)-6,6-dimethyl-tetrahydrothiopyran-2,4-dion als rotes Oel vom Brechungsindex $n_D^{22,8}$ : 1,5358.

Herstellung der Ausgangsverbindung

a)

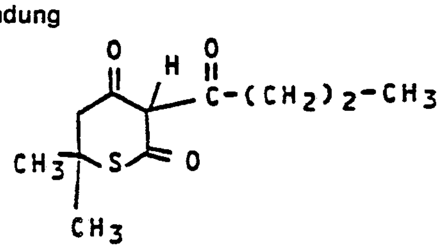

27,1 g (0,12 Mol) 6,6-Dimethyl-4-butyryloxy-5,6-dihydro-α-thiopyron und 0,01 g (0,1 mMol) 4-(N,N-Dimethylamino)-pyridin werden in 150 ml Toluol so lange zum Sieden erhitzt (ca. 3 Stunden), bis die dünnschichtchromatographische Analyse (Kieselgel/Dichlormethan oder Chloroform + 2 % Methanol) vollständigen Umsatz des Ausgangsmateriales anzeigt. (Das Produkt ergibt beim Behandeln mit wäßriger Fe(III)-Chlorid-Lösung eine intensiv orangerote Färbung). Zur Aufarbeitung wäscht man mit verdünnter Salzsäure, trocknet über Magnesiumsulfat und entfernt das Lösungsmittel im Vakuum. Man erhält 15,1 g (55 % der Theorie) an 6,6-Dimethyl-3-butyryltetrahydrothiopyran-2,4-dion als Oel.

b)

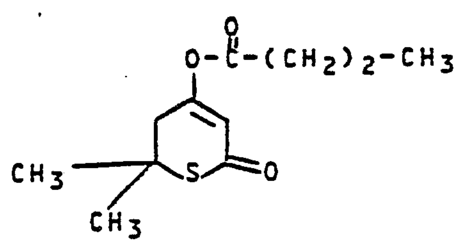

20 g (0,12 Mol) 6,6-Dimethyltetrahydrothiopyran-2,4-dion und 19,9 g (0,13 Mol) Diazabicycloundecen (DBU) in 240 ml Toluol werden bei 0 °C unter Rühren tropfenweise mit 13,9 g (0,13 Mol) Butyrylchlorid versetzt, nach beendeter Zugabe weitere 2 Stunden bei 0 °C gerührt und mit 120 ml Wasser versetzt. Die organische Phase wird abgetrennt, die wäßrige Phase noch zweimal mit Toluol extrahiert, die vereinigten organischen Phasen mit 5 %iger Salzsäure und mit gesättigter wäßriger Natriumchloridlösung gewaschen, über Magnesiumsulfat getrocknet und im Vakuum vom Lösungsmittel befreit. Man erhält 27,1 g (93,5 % der Theorie) an 6,6-Dimethyl-4-butyryloxy-5,6-dihydro-α-thiopyron als Oel, welches ohne weitere Reinigung in die nächste Stufe eingesetzt werden kann.

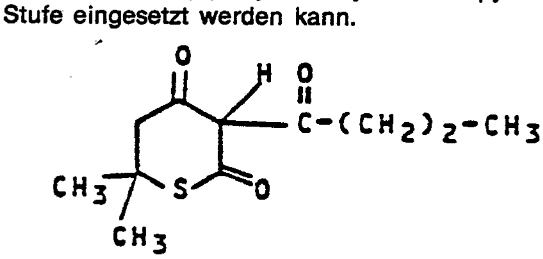

(alternative Herstellung)

5 g (0,035 Mol) 6,6-Dimethyltetrahydrothiopyran-2,4-dion in 100 ml Pyridin werden zunächst mit 9,6 g (0,07 Mol) Zinkchlorid und danach tropfenweise mit 3,7 g (0,03 Mol) Buttersäurechlorid versetzt. Nach beendeter Zugabe erhitzt man für 4 Stunden auf Rückflußtemperatur, gießt die abgekühlte Reaktionsmischung in 200 ml halbkonzentrierte Salzsäure, extrahiert mehrfach mit Dichlormethan, trocknet die vereinigten organischen Phasen über Magnesiumsulfat und entfernt das Lösungsmittel im Vakuum. Man erhält 2,9 g (36,6 % der Theorie) an 6,6-Dimethyl-3-butyryltetrahydrothiopyran-2,4-dion als Oel.

In entsprechender Weise und gemäß den allgemeinen Herstellungsangaben erhält man die folgenden Tetrahydrothiopyran-2,4-dione der allgemeinen Formel (I)

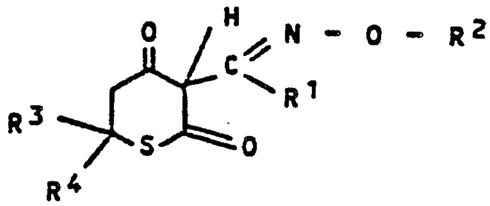

(I)

Tabelle 2

| Bsp.Nr. | $R^1$ | $R^2$ | $R^3$ | $R^4$ | Schmelzpunkt/ Brechungsindex |
|---|---|---|---|---|---|
| 2 | $CH_3-(CH_2)_2-$ | $C_2H_5-$ | $CH_3-$ | $CH_3-$ | $n_D^{23,6}$:1,5390 |
| 3 | $CH_3-(CH_2)_2-$ | $(CH_3)_2CH-$ | $CH_3-$ | $CH_3-$ | $n_D^{22,8}$:1,5317 |
| 4 | 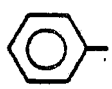 | $C_2H_5$ | $CH_3$ | $CH_3$ | Fp. 78-81°C |
| 5 | 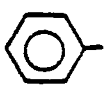 | $CH_2=CH-CH_2-$ | $CH_3$ | $CH_3$ | Fp. 119-122°C |
| 6 | $CH_3-(CH_2)_2-$ | $CH_2-CH=CHCl$ | $CH_3$ | $CH_3$ | $n_D^{22}$:1,5509 |

## Anwendungsbeispiele

In den folgenden Anwendungsbeispielen wurde die nachstehend aufgeführte Verbindung als Vergleichsbeispiel herangezogen :

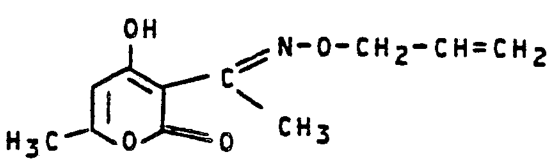

(A)

3-(1-Allyloximino-ethyl)-4-hydroxy-6-methyl-α-pyron
(vergl. z. B. DE-AS 2 439 104).

## Beispiel A

Pre-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

Eine deutliche Überlegenheit in der Wirksamkeit gegenüber dem Stand der Technik zeigt bei diesem Test z. B. die Verbindung gemäß Herstellungsbeispiel 1.

Beispiel B

Post-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 2 000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten :

0 % = keine Wirkung (wie unbehandelte Kontrolle)
100 % = totale Vernichtung

Eine deutliche Überlegenheit in der Wirksamkeit gegenüber dem Stand der Technik zeigt bei diesem Test z. B. die Verbindung gemäß Herstellungsbeispiel 1.

**Patentansprüche**

1. Substituierte Tetrahydrothiopyran-2,4-dione der allgemeinen Formel (I),

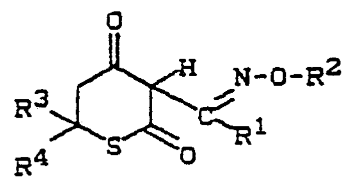

(I)

in welcher

$R^1$ für Wasserstoff, Methyl, Ethyl, n- oder i-Propyl, n-, i-, s- oder t-Butyl, n- oder i-Pentyl, n- oder i-Hexyl, Vinyl, Allyl, n- oder i-Butenyl, n- oder i-Pentenyl, n- oder i-Hexenyl, Propargyl, Methylpropargyl, Dimethylpropargyl, Methoxymethyl, Ethoxymethyl, Isopropoxymethyl, Methoxyethyl, Ethoxyethyl, Methylthiomethyl, Methylthioethyl, Ethylthioethyl, Trifluormethyl, Dichlorfluormethyl, Difluorchlormethyl, Trichlormethyl, Dichlormethyl, Chlormethyl, 2-Chlorethyl, 2-Bromethyl, 2,2,2-Trichlorethyl, 2,2,2-Trifluorethyl, Cyclopropyl, Cyclobutyl, Cyclopentyl, Cyclohexyl oder gegebenenfalls ein- bis dreifach, gleich oder verschieden durch Fluor, Chlor, Brom, Cyano, Nitro, Methyl, Methoxy oder Trifluormethyl substituiertes phenyl steht,

$R^2$ für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, Methoxymethyl, Ethoxymethyl, Isopropoxymethyl, Methoxyethyl, Ethoxyethyl, Methylthiomethyl, Methylthioethyl, Ethylthioethyl, 2-Chlorethyl, 2-Bromethyl, 2,2,2-Trifluorethyl, 2,2,2-Trichlorethyl, 3-Chlorpropenyl, Methoxycarbonylmethyl, Ethoxycarbonylmethyl, Methoximinomethyl, Methoximinoethyl, Methoximino-2-propyl, gegebenenfalls ein- bis dreifach, gleich oder

verschieden durch Fluor, Chlor, Brom, Cyano, Nitro, Methyl, Methoxy oder Trifluormethyl substituiertes Benzyl oder für einen gegebenenfalls ein- bis dreifach, gleich oder verschieden durch Chlor, Methyl oder Trifluormethyl substituierten über eine Methylenbrücke verknüpften Heterocyclus der Formel

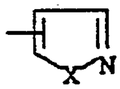 ; 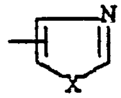 ; 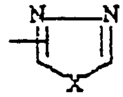 oder 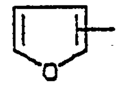

steht, wobei X jeweils für Sauerstoff oder Schwefel steht, und

$R^3$ und $R^4$ unabhängig voneinander jeweils für Wasserstoff, Methyl, Ethyl, n- oder i-Propyl, n-, i-, s- oder t-Butyl, Methoxymethyl, Ethoxymethyl, Methoxyethyl, Ethoxyethyl, Methylthiomethyl, Ethylthiomethyl, Ethylthioethyl, Ethylthiopropyl, Cyclopentyl, Cyclohexyl oder für jeweils gegebenenfalls ein- bis dreifach, gleich oder verschieden durch Fluor, Chlor, Brom, Cyano, Nitro, Methyl, Methoxy, Ethoxy, Methylthio, Ethylthio oder Trifluormethyl substituiertes Phenyl, Benzyl, Phenylethyl, Phenoxymethyl oder Phenoxyethyl stehen.

2. Verfahren zur Herstellung von substituierten Tetrahydrothiopyran-2,4-dionen der allgemeinen Formel (I),

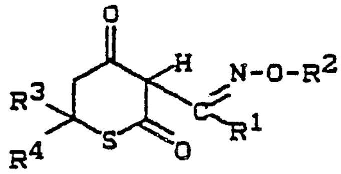

(I)

in welcher

$R^1$ für Wasserstoff, Methyl, Ethyl, n- oder i-Propyl, n-, i-, s- oder t-Butyl, n- oder i-Pentyl, n- oder i-Hexyl, Vinyl, Allyl, n- oder i-Butenyl, n- oder i-Pentenyl, n- oder i-Hexenyl, Propargyl, Methylpropargyl, Dimethylpropargyl, Methoxymethyl, Ethoxymethyl, Isopropoxymethyl, Methoxyethyl, Ethoxyethyl, Methylthiomethyl, Methylthioethyl, Ethylthioethyl, Trifluormethyl, Dichlorfluormethyl, Difluorchlormethyl, Trichlormethyl, Dichlormethyl, Chlormethyl, 2-Chlorethyl, 2-Bromethyl, 2,2,2-Trichlorethyl, 2,2,2-Trifluorethyl, Cyclopropyl, Cyclobutyl, Cyclopentyl, Cyclohexyl oder gegebenenfalls ein- bis dreifach, gleich oder verschieden durch Fluor, Chlor, Brom, Cyano, Nitro, Methyl, Methoxy oder Trifluormethyl substituiertes Phenyl steht,

$R^2$ für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, Methoxymethyl, Ethoxymethyl, Isopropoxymethyl, Methoxyethyl, Ethoxyethyl, Methylthiomethyl, Methylthioethyl, Ethylthioethyl, 2-Chlorethyl, 2-Bromethyl, 2,2,2,-Trifluorethyl, 2,2,2-Trichlorethyl, 3-Chlorpropenyl, Methoxycarbonylmethyl, Ethoxycarbonylmethyl, Methoximinomethyl, Methoximinoethyl, Methoximino-2-propyl, gegebenenfalls ein- bis dreifach, gleich oder verschieden durch Fluor, Chlor, Brom, Cyano, Nitro, Methyl, Methoxy oder Trifluormethyl substituiertes Benzyl oder für einen gegebenenfalls ein- bis dreifach, gleich oder verschieden durch Chlor, Methyl oder Trifluormethyl substituierten über eine Methylenbrücke verknüpften Heterocyclus der Formel

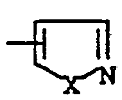 ; 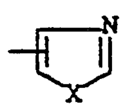 ; 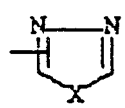 oder 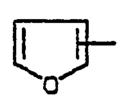

steht, wobei X jeweils für Sauerstoff oder Schwefel steht, und

$R^3$ und $R^4$ unabhängig voneinander jeweils für Wasserstoff, Methyl, Ethyl, n- oder i-Propyl, n-, i-, s- oder t-Butyl, Methoxymethyl, Ethoxymethyl, Methoxyethyl, Ethoxyethyl, Methylthiomethyl, Ethylthiomethyl, Ethylthioethyl, Ethylthiopropyl, Cyclopentyl, Cyclohexyl oder für jeweils gegebenenfalls ein- bis dreifach, gleich oder verschieden durch Fluor, Chlor, Brom, Cyano, Nitro, Methyl, Methoxy, Ethoxy, Methylthio, Ethylthio oder Trifluormethyl substituiertes Phenyl, Benzyl, Phenylethyl, Phenoxymethyl oder Phenoxyethyl stehen,

dadurch gekennzeichnet, daß man Aldehyde bzw. Ketone der allgemeinen Formel (II),

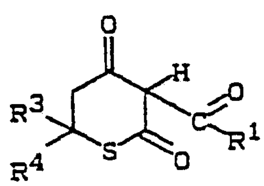

(II)

in welcher $R^1$, $R^3$ und $R^4$ die oben angegebene Bedeutung haben, mit Hydroxylamin-Derivaten der Formel (III),

$$H_2N—O—R^2 \qquad (III)$$

in welcher $R^2$ die oben angegebene Bedeutung hat, oder mit deren Hydrosalzen, gegebenenfalls in Gegenwart eines Verdünnungsmittels und gegebenenfalls in Gegenwart eines Säurebindemittels, umsetzt.

3. Herbizide Mittel, gekennzeichnet durch einen Gehalt an mindestens einem substituierten Tetrahydrothiopyran-2,4-dion der Formel (I) gemäß Anspruch 1 und 2.

4. Verwendung von substituierten Tetrahydrothiopyran-2,4-dionen der Formel (I) gemäß Ansprüche 1 und 2 zur Bekämpfung von unerwünschtem Pflanzenwachstum.

5. Verfahren zur Herstellung von herbiziden Mitteln, dadurch gekennzeichnet, daß man substituierte Tetrahydrothiopyran-2,4-dione der Formel (I) gemäß Ansprüche 1 und 2 mit Streckmitteln und/oder oberflächenaktiven Mitteln vermischt.

6. Verbindungen der Formel (II)

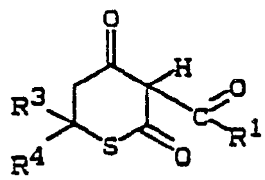

(II)

in welcher $R^1$, $R^3$ und $R^4$ die in Anspruch 1 angegebene Bedeutung haben.

7. Verfahren zur Herstellung von Verbindungen der Formel (II),

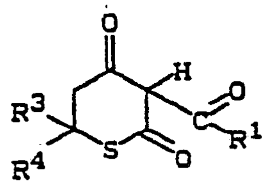

(II)

in welcher $R^1$, $R^3$ und $R^4$ die in Anspruch 2 angegebene Bedeutung haben, dadurch gekennzeichnet, daß man Tetrahydrothiopyran-2,4-dione der Formel (IV)

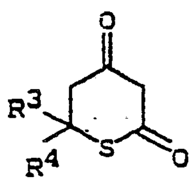

(IV)

in welcher $R^3$ und $R^4$ die in Anspruch 2 angegebene Bedeutung haben, mit Acylierungsmitteln der Formel (V),

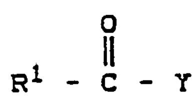

(V)

in welcher

$R^1$ die in Anspruch 2 angegebene Bedeutung hat und

Y für eine elektronenanziehende Abgangsgruppe, insbesondere für Halogen oder den Rest

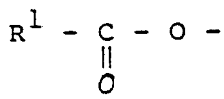

steht, wobei $R^1$ die in Anspruch 2 angegebene Bedeutung hat, zunächst in einer ersten Stufe, gegebenenfalls in Gegenwart eines Verdünnungsmittels und gegebenenfalls in Gegenwart eines Säurebindemittels, bei Temperaturen zwischen — 20 °C und + 50 °C umsetzt und die so erhältlichen O-Acylderivate der Formel (VI),

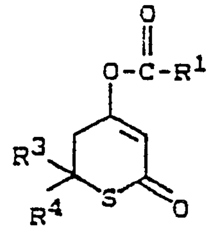

(VI)

in welcher $R^1$, $R^3$ und $R^4$ die in Anspruch 2 angegebene Bedeutung haben, in einer 2. Stufe, ebenfalls gegebenenfalls in Gegenwart eines Verdünnungsmittels und in Gegenwart eines basischen Katalysators, bei Temperaturen zwischen 0 °C und + 120 °C umlagert.

**Claims**

1. Substituted tetrahydrothiopyran-2,4-diones of the general formula (I)

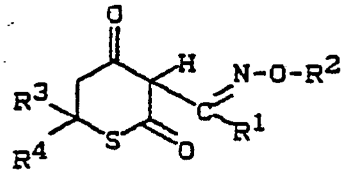

(I)

in which

$R^1$ represents hydrogen, methyl, ethyl, n- or i-propyl, n-, i-, s- or t-butyl, n- or i-pentyl, n- or i-hexyl, vinyl, allyl, n- or i-butenyl, n- or i-pentenyl, n- or i-hexenyl, propargyl, methylpropargyl, dimethylpropargyl, methoxymethyl, ethoxymethyl, isopropoxymethyl, methoxyethyl, ethoxyethyl, methylthiomethyl, methylthioethyl, ethylthioethyl, trifluoromethyl, dichlorofluoromethyl, difluorochloromethyl, trichloromethyl, dichloromethyl, chloromethyl, 2-chloroethyl, 2-bromoethyl, 2,2,2-trichloroethyl, 2,2,2-trifluoroethyl, cyclopropyl, cyclobutyl, cyclopentyl, or cyclohexyl or represents phenyl which is optionally monosubstituted, disubstituted or trisubstituted by identical or different substituents from the series comprising fluorine, chlorine, bromine, cyano, nitro, methyl, methoxy or trifluoromethyl,

$R^2$ represents methyl, ethyl, n- or i-propyl, n-, i-, s- or t-butyl, n- or i-pentyl, n- or i-hexyl, allyl, n- or i-butenyl, n- or i-pentenyl, propargyl, methoxymethyl, ethoxymethyl, isopropoxymethyl, methoxyethyl, ethoxyethyl, methylthiomethyl, methylthioethyl, ethylthioethyl, 2-chloroethyl, 2-bromoethyl, 2,2,2-trifluoroethyl, 2,2,2-trichloroethyl, 3-chloropropenyl, methoxycarbonylmethyl, ethoxycarbonylmethyl, methoxyiminomethyl, methoxyiminoethyl or methoxyimino-2-propyl, or represents benzyl which is optionally monosubstituted, disubstituted or trisubstituted by identical or different substituents from the series comprising fluorine, chlorine, bromine, cyano, nitro, methyl, methoxy or trifluoromethyl, or represents a heterocyclic ring of the formula

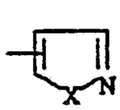 ; 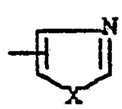 ; 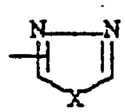 or 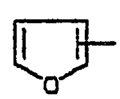

where X in each case stands for oxygen or sulphur, which is optionally monosubstituted, disubstituted or trisubstituted by identical or different substituents from the series comprising chlorine, methyl or trifluoromethyl, and which is bonded via a methylene bridge, and

$R^3$ and $R^4$ each independently of one another represent hydrogen, methyl, ethyl, n- or i-propyl, n-, i-, s- or t-butyl, methoxymethyl, ethoxymethyl, methoxyethyl, ethoxyethyl, methylthiomethyl, ethylthiomethyl, ethylthioethyl, ethylthiopropyl, cyclopentyl or cyclohexyl, or represents phenyl, benzyl, phenylethyl, phenoxymethyl or phenoxyethyl, each optionally monosubstituted, disubstituted or trisubstituted by identical or different substituents from the series comprising fluorine, chlorine, bromine, cyano, nitro, methyl, methoxy, ethoxy, methylthio, ethylthio or trifluoromethyl.

2. Process for the preparation of substituted tetrahydrothiopyran-2,4-diones of the general formula (I)

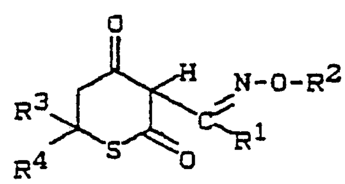

(I)

in which

$R^1$ represents hydrogen, methyl, ethyl, n- or i-propyl, n-, i-, s- or t-butyl, n- or i-pentyl, n- or i-hexyl, vinyl, allyl, n- or i-butenyl, n- or i-pentenyl, n- or i-hexenyl, propargyl, methylpropargyl, dimethylpropargyl, methoxymethyl, ethoxymethyl, isopropoxymethyl, methoxyethyl, ethoxyethyl, methylthiomethyl, methylthioethyl, ethylthioethyl, trifluoromethyl, dichlorofluoromethyl, difluorochloromethyl, trichoromethyl, dichloromethyl, chloromethyl, 2-chloroethyl, 2-bromoethyl, 2,2,2-trichloroethyl, 2,2,2-trifluoroethyl, cyclopropyl, cyclobutyl, cyclopentyl, or cyclohexyl or represents phenyl which is optionally monosubstituted, disubstituted or trisubstituted by identical or different substituents from the series comprising fluorine, chlorine, bromine, cyano, nitro, methyl, methoxy or trifluoromethyl,

$R^2$ represents methyl, ethyl, n- or i-propyl, n-, i-, s- or t-butyl, n- or i-pentyl, n- or i-hexyl, allyl, n- or i-butenyl, n- or i-pentenyl, propargyl, methoxymethyl, ethoxymethyl, isopropoxymethyl, methoxyethyl, ethoxyethyl, methylthiomethyl, methylthioethyl, ethylthioethyl, 2-chloroethyl, 2-bromoethyl, 2,2,2-trifluoroethyl, 2,2,2-trichloroethyl, 3-chloropropenyl, methoxycarbonylmethyl, ethoxycarbonylmethyl, methoxyiminomethyl, methoxyiminoethyl or methoxyimino-2-propyl, or represents benzyl which is optionally monosubstituted, disubstituted or trisubstituted by identical or different substituents from the series comprising fluorine, chlorine, bromine, cyano, nitro, methyl, methoxy or trifluoromethyl, or represents a heterocyclic ring of the formula

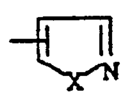 ; 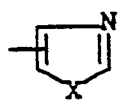 ; 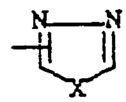 or 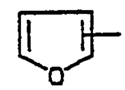

where X in each case stands for oxygen or sulphur, which is optionally monosubstituted, disubstituted or trisubstituted by identical or different substituents from the series comprising chlorine, methyl or trifluoromethyl, and which is bonded via a methylene bridge, and

$R^3$ and $R^4$ each independently of one another represent hydrogen, methyl, ethyl, n- or i-propyl, n-, i-, s- or t-butyl, methoxymethyl, ethoxymethyl, methoxyethyl, ethoxyethyl, methylthiomethyl, ethylthiomethyl, ethylthioethyl, ethylthiopropyl, cyclopentyl or cyclohexyl, or represents phenyl, benzyl, phenylethyl, phenoxymethyl or phenoxyethyl, each optionally monosubstituted, disubstituted or trisubstituted by identical or different substitutents from the series comprising fluorine, chlorine, bromine, cyano, nitro, methyl, methoxy, ethoxy, methylthio, ethylthio or trifluoromethyl,

characterized in that aldehydes or ketones of the general formula (II)

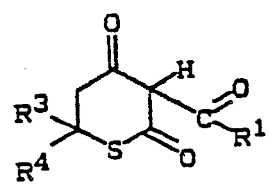

(II)

in which $R^1$, $R^3$ and $R^4$ have the meaning given above, are reacted with hydroxylamine derivatives of the formula (III)

$$H_2N—O—R^2 \qquad (III)$$

in which $R^2$ has the meaning given above, or with their hydro salts, if appropriate in the presence of a diluent and, if appropriate, in the presence of an acid-binding agent.

3. Herbicidal agents, characterized in that they contain at least one substituted tetrahydrothiopyran-2,4-dione of the formula (I) according to Claims 1 and 2.

4. Use of substituted tetrahydrothiopyran-2,4-diones of the formula (I) according to Claims 1 and 2 for combating undesired plant growth.

5. Process for the preparation of herbicidal agents, characterized in that substituted tetrahydrothiopyran-2,4-diones of the formula (I) according to Claims 1 and 2 are mixed with extenders and/or surface-active agents.

6. Compounds of the formula (II)

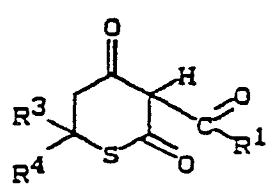

(II)

in which $R^1$, $R^3$ and $R^4$ have the meaning given in Claim 1.

7. Process for the preparation of compounds of the formula (II)

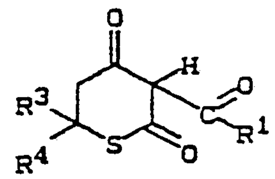

(II)

in which $R^1$, $R^3$ and $R^4$ have the meaning given in Claim 2, characterized in that tetrahydrothiopyran-2,4-diones of the formula (IV)

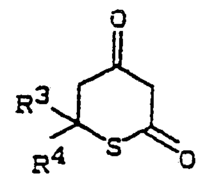

(IV)

in which $R^3$ and $R^4$ have the meaning given in Claim 2, are reacted with acylating agents of the formula (V)

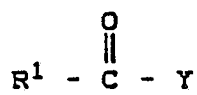

(V)

in which
$R^1$ has the meaning given in Claim 2 and
Y represents an electron-attracting leaving group, in particular halogen or the radical

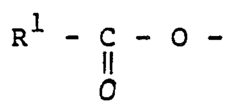

wherein $R^1$ has the meaning given in Claim 2, the reaction first being carried out in a first stage, if appropriate in the presence of a diluent and, if appropriate, in the presence of an acid-binding agent, at temperatures between — 20 °C and + 50 °C, and the resulting 0-acyl derivatives of the formula (VI)

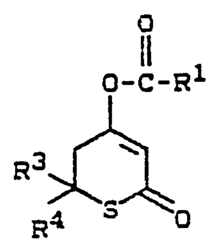

(VI)

in which $R^1$, $R^3$ and $R^4$ have the meaning given in Claim 2, being subjected to a rearrangement reaction in a 2nd stage, if appropriate likewise in the presence of a diluent and in the presence of a basic catalyst at temperatures between 0 °C and + 120 °C.

**Revendications**

1. Tétrahydrothiopyrane-2,4-diones substituées répondant à la formule générale (I),

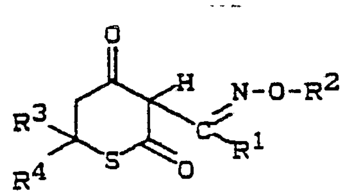

(I)

dans laquelle
$R^1$ représente de l'hydrogène, un radical méthyle, éthyle, n- ou i-propyle, n-, i-, s- ou t-butyle, n- ou i-pentyle, n- ou i-hexyle, vinyle, allyle, n- ou i-butenyle, n- ou i-pentenyle, n- ou i-hexényle, propargyle, méthylpropargyle, diméthylpropargyle, méthoxyméthyle, éthoxyméthyle, isopropoxyméthyle, méthoxyéthyle, éthoxyéthyle, méthylthiométhyle, méthylthioéthyle, éthylthioéthyle, trifluorométhyle, dichlorofluorométhyle, difluorochlorométhyle, trichlorométhyle, dichlorométhyle, chlorométhyle, 2-chloréthyle, 2-brométhyle, 2,2,2-trichloréthyle, 2,2,2-trifluoréthyle, cyclopropyle, cyclobutyle, cyclopentyle, cyclohexyle ou phényle, substitués éventuellement 1 à 3 fois de manière identique ou différente par le fluor, le chlore, le brome, un radical cyano, nitro, méthyle, méthoxy ou trifluorométhyle

$R^2$ représente un groupe méthyle, éthyle, n- ou i-propyle, n-, i-, s- ou t-butyle, n- ou i-pentyle, n- ou i-hexyle, allyle, n- ou i-butenyle, n- ou i-pentenyle, propargyle, méthoxyméthyle, éthoxyméthyle, isopropoxyméthyle, méthoxyéthyle, éthoxyéthyle, méthylthiométhyle, méthylthioéthyle, éthylthioéthyle, 2-chloréthyle, 2-brométhyle, 2,2,2-trifluoréthyle, 2,2,2-trichloréthyle, 3-chloropropényle, méthoxycarbonylméthyle, éthoxycarbonylméthyle, méthoximinométhyle, méthoximinoéthyle, méthoximino-2-propyle, benzyle substitué éventuellement une à trois fois de manière identique ou différente par le fluor, le chlore, le brome, un radical cyano, nitro, méthyle, méthoxy ou trifluorométhyle ou un composé hétérocyclique répondant à la formule

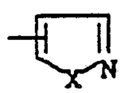 ; 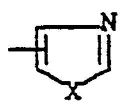 ; 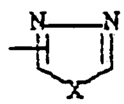 ou 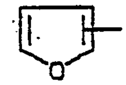

lié par un pont de méthylène et substitué éventuellement une à trois fois, de manière identique ou différente par le chlore, le méthyle ou le trifluorométhyle où X représente chaque fois de l'oxygène ou du soufre, et

$R^3$ et $R^4$ représentent, indépendamment l'un de l'autre, de l'hydrogène, un radical méthyle, éthyle, n- ou i-propyle, n-, i-, s- ou t-butyle, méthoxyméthyle, éthoxyméthyle, méthoxyéthyle, éthoxyéthyle, méthylthiométhyle, éthylthiométhyle, éthylthioéthyle, éthylthiopropyle, cyclopentyle, cyclohexyle ou un radical phényle, benzyle, phényléthyle, phénoxyméthyle ou phénoxyéthyle, éventuellement substitués chacun une à trois fois, de manière identique ou différente par le fluor, le chlore, le brome, le radical cyano, nitro, méthyle, méthoxy, éthoxy, méthylthio, éthylthio ou trifluorométhyle.

2. Procédé de production de tétrahydrothiopyrane-2,4-diones substituées répondant à la formule générale (I),

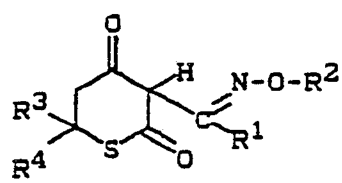

(I)

dans laquelle

$R^1$ représente de l'hydrogène, un radical méthyle, éthyle, n- ou i-propyle, n-, i-, s- ou t-butyle, n- ou i-pentyle, n- ou i-hexyle, vinyle, allyle, n- ou i-butényle, n- ou i-pentenyle, n- ou i-hexényle, propargyle, méthylpropargyle, diméthylpropargyle, méthoxyméthyle, éthoxyméthyle, isopropoxyméthyle, méthoxyéthyle, éthoxyéthyle, méthylthiométhyle, méthylthioéthyle, éthylthioéthyle, trifluorométhyle, dichlorofluorométhyle, difluorochlorométhyle, trichlorométhyle, dichlorométhyle, chlorométhyle, 2-chloréthyle, 2-brométhyle, 2,2,2-trichloréthyle, 2,2,2-trifluoréthyle, cyclopropyle, cyclobutyle, cyclopentyle, cyclohexyle ou phényle éventuellement substitué une à trois fois, de manière identique ou différente, par le fluor, le chlore, le brome, un radical cyano, nitro, méthyle, méthoxy ou trifluorométhyle,

$R^2$ représente un radical méthyle, éthyle, n- ou i-propyle, n-, i-, s- ou t-butyle, n- ou i-pentyle, n- ou i-hexyle, allyle, n- ou i-butényle, n- ou i-pentényle, propargyle, méthoxyméthyle, éthoxyméthyle, isopropoxyméthyle, méthoxyéthyle, éthoxyéthyle, méthylthiométhyle, méthylthioéthyle, éthylthioéthyle, 2-chloréthyle, 2-brométhyle, 2,2,2-trifluoréthyle, 2,2,2-trichloréthyle, 3-chloropropényle, méthoxycarbonylméthyle, éthoxycarbonylméthyle, méthoximinométhyle, méthoximinoéthyle, méthoximino-2-propyle, un radical benzyle éventuellement substitué une à trois fois, de manière identique ou différente, par le fluor, le chlore, le brome, le radical cyano, nitro, méthyl, méthoxy ou trifluorométhyle, un composé hétérocyclique répondant à la formule

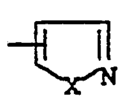 ; 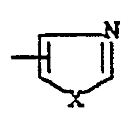 ; 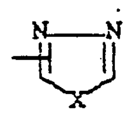 ou 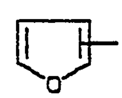

lié par un pont de méthylène et éventuellement substitué une à trois fois de manière identique ou différente par le chlore, le méthyle ou le trifluorométhyle, où X représente chaque fois de l'oxygène ou du soufre et

$R^3$ et $R^4$ représentent, indépendamment l'un de l'autre, de l'hydrogène, un radical méthyle, éthyle, n- ou i-propyle, n-, i-, s-, ou t-butyle, méthoxyméthyle, éthoxyméthyle, méthoxyéthyle, éthoxyéthyle, méthylthiométhyle, éthylthiométhyle, éthylthioéthyle, éthylthiopropyle, cyclopentyle, cyclohexyle ou un radical phényle, benzyle, phényléthyle, phénoxyméthyle ou phénoxyéthyle, éventuellement substitués chacun une à trois fois, de manière identique ou différente par le fluor, le chlore, le brome, le radical cyano, nitro, méthyle, méthoxy, éthoxy, méthylthio, éthylthio ou trifluorométhyle.
caractérisé en ce que l'on transforme des aldéhydes ou des cétones répondant à la formule générale (II)

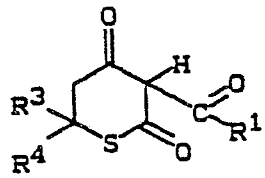

(II)

dans laquelle $R^1$, $R^3$ et $R^4$ ont les significations mentionnées ci-dessus, par des dérivés de l'hydroxylamine répondant à la formule (III),

$$H_2N—O—R^2 \quad \text{(III)}$$

dans laquelle $R^2$ a la signification mentionnée ci-dessus, ou par leurs sels hydratés, éventuellement en présence d'un diluant et éventuellement en présence d'un agent fixant les acides.

3. Agent herbicide, caractérisé par une teneur en au moins une tétrahydrothiopyrane-2,4-dione substituée répondant à la formule (I) et selon les revendications 1 et 2.

4. Utilisation de tétrahydrothiopyrane-2,4-diones substituées répondant à la formule (I) et selon la revendication 1 et 2, pour combattre les végétaux indésirables.

5. Procédé pour la production d'agents herbicides caractérisé en ce que l'on mélange des tétrahydrothiopyrane-2,4-diones substituées répondant à la formule (I) et selon les revendications 1 et 2, à des agents de dilution et/ou à des agents tensio-actifs.

6. Composés de la formule (II)

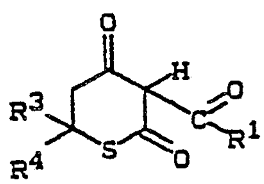

(II)

dans lesquels $R^1$, $R^3$ et $R^4$ ont la signification mentionnée à la revendication 1.

7. Procédé de production de composés répondant à la formule (II)

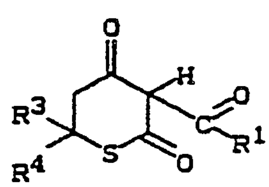

(II)

dans laquelle $R^1$, $R^3$ et $R^4$ ont la signification indiquée à la revendication 2, caractérisé en ce que l'on transforme une tétrahydrothiopyrane-2,4-dione répondant à la formule (IV)

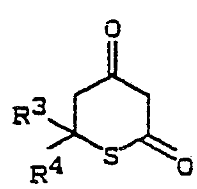

(IV)

dans laquelle $R^3$ et $R^4$ ont les significations mentionnées à la revendication 2, par un agent d'acylation répondant à la formule (V)

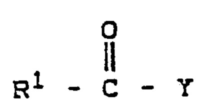

(V)

dans laquelle

$R^1$ a la signification indiquée à la revendication 2 et Y représente un groupe labile capteur d'électrons et, en particulier, un halogène ou un résidu

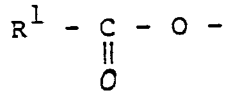

tandis que $R^1$ a la signification indiquée à la revendication 2, en le traitant tout d'abord, dans une première étape, éventuellement en présence d'un diluant et éventuellement en présence d'un agent fixant les acides à des températures comprises entre — 20 °C et + 50 °C, et que le dérivé O-acylique ainsi obtenu et répondant à la formule (VI)

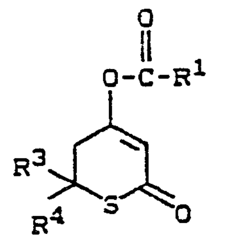

(VI)

dans laquelle $R^1$, $R^3$ et $R^4$ ont les significations mentionnées à la revendication 2 est transformé au cours d'une seconde phase, éventuellement aussi en présence d'un diluant et en présence d'un catalyseur basique, à des températures comprises entre 0 °C et + 120 °C.